# EUROPEAN PATENT APPLICATION

(11) **EP 3 482 756 A1**
(43) Date of publication of application: **15.05.2019**
(21) Application number: 18461625.8
(22) Date of filing: 10.11.2018
(51) Int. Cl.: A61K 31/202, A61K 31/455, A61P 3/06

(54) **PHARMACEUTICAL COMPOSITION AND USE OF THE PHARMACEUTICAL COMPOSITION**

(30) Priority: 10.11.2017 PL 42341517
(71) Applicant: Lambdafin Sp. z o.o., 61-770 Poznan (PL)
(72) Inventor: Rze niczak, Janusz, 60-465 Pozna (PL); Burchardt, Pawe, 62-002 Z otniki (PL)
(74) Representative: Witek, Rafal

(57) **Abstract**

A pharmaceutical composition with ethyl ester of eicosapentaenoic acid (EPA) and ethyl ester of docosahexaenoic acid (DHA), consists of ingredient A, which is a mixture of ethyl ester of eicosapentaenoic acid (EPA) and ethyl ester of docosahexaenoic acid (DHA) in mass ratio of 1 : 0.6 ÷ 1.0, and ingredient B, which is nicotinic acid amide, in mass ratio to the ethyl ester of eicosapentaenoic acid (EPA) of 1 : 0.1 ÷ 2, within a daily dose range for nicotinic acid amide between 25 mg - 500 mg, for simultaneous administration.

The object of the invention is also use of the pharmaceutical composition for primary and secondary prevention of cardiovascular events.

## Description

The object of the present invention is a pharmaceutical composition and use of the pharmaceutical composition for prevention of primary and secondary cardiovascular events.

It is estimated that in 2008, 17.3 million people with cardiovascular diseases (CVD) died, which constitutes 30% of all deaths in the world (WHO 2016). Within this group, around 7.3 million people are patients with ischemic heart disease (coronary heart disease, CHD). Ischemic heart disease is in 98% of cases caused by atherosclerosis. The atherosclerotic process is caused by a local inflammation encompassing the whole "thickness" of an arterial blood vessel. Over the course of this process, an atherosclerotic plaque is formed. The substrate for the plaque formation are lipid compounds transported and delivered to the wall of the vessel, where over the course of the ongoing inflammation they undergo significant modifications (oxidation, glycosylation). Cardiological recommendations, both in secondary prevention of cardiovascular diseases as well as in primary one, namely for subjects not yet suffering from atherosclerosis, determine the requirement of eliminating all known conditions promoting atherosclerosis progression. The most important include reducing plasma concentrations of lipid substrates: low density lipoproteins (LDL), total cholesterol and triglycerides (TG) /included in chylomicrons and very low density lipoproteins (VLDL). It is logical that in order to reduce the atherosclerotic process all of the clinical states that may induce or aggravate inflammation and damages of arterial blood vessel walls should also be altered. One of the abovementioned states, influencing an increase in the atherosclerotic process, is a carbohydrate metabolism disorder, known in the field as glucose intolerance and/or diabetes. Diabetes is a carbohydrate, lipid and protein metabolism disorder caused by a deficiency or an aberrant activity of insulin /Eur Heart J, 2007; 28: 2375-2414/. Insulin deficiency or a developing insulin resistance after many years may lead to micro-, macroangiopathy, neuropathy, nephropathy and retinopathy. At the molecular level this is explained through an activation of the polyol pathway, chronic protein glycosylation or an elevated oxidative stress. Macro- and microangiopathy are a consequence of a disruption in vascular homeostasis, by a plasma lipoprotein metabolism disorder among others. The plasma lipoprotein metabolism disorder in diabetes have a qualitative and quantitative nature. In type 1 diabetes (T1DM), changes in the ratio and type of the transported lipid fractions prevail, arising from an antagonistic activity of insulin on a system of enzymes and other functional proteins regulating lipoprotein transformations. In type 2 diabetes (T2DM) caused by insulin resistance, the additional element affecting lipid metabolism is excessively developed visceral fat tissue, being the source of substrates for synthesis of endogenous lipoproteins. Lipid metabolism disorder, regardless of the etiology of diabetes, manifests itself as a systemic increase of the triglyceride fraction (TG), because postprandial hyperchylomicronemia and an increase in liver synthesis of very low density lipoproteins (VLDLs) are observed /Arterioscler Thromb Vasc Biol, 2008; 28: 1225-1236, Curr Diab Rep, 2008; 8: 60-64/. There are several causes of the increased endogenous formation of VLDLs. First of all, an activation of 'de novo' lipogenesis in hepatocytes is involved /Arterioscler Thromb Vasc Biol, 2008; 28: 1225-1236, Obesity, 2006; 14: 41S-49S/, which is a consequence of a disruption in regulation of this process. That is because an imbalance occurs between the insulin-induced sterol response element /SREBP//Horm Res, 2007; 68: 72-82/ and the glucose response element/CHREB/, the activity of the latter increasing with insulin resistance /Endocr J, 2008; 55: 617-624/.

Secondly, insulin deficiency promotes excessive delivery of TGs and free fatty acids (FFAs) from fat tissue (FT) to hepatocytes, that being an effect of a forced activity of the hormone-sensitive lipase and inhibition of lipogenesis in FT /Kardiol Pol, 2008; 66: 1215-1220/. Thirdly, the inhibitory effect of insulin on the expression of MTP (microsomal transfer protein) /Diabetes Metab, 2005; 31: 429-439/ phospholipase D and ARF-1 (adenosine response factor), is abolished, those being the proteins mediating VLDL biosynthesis /Diabetes Metab, 2005; 31: 429-439/. These proteins are consecutively involved in incorporating triglycerides into apoprotein B100 (apoB100), in pre-VLDL and conversion thereof into VLDL in the endoplasmic reticulum of hepatocytes /Diabetes Metab, 2005; 31: 429-439/.

Lipid disorders in states of insulin resistance are also associated with a reduced activity of lipoprotein lipase (LPL) /Diabetes Metab, 2005; 31:429-439/. The consequence of this is deficient hydrolysis of the postprandially formed chylomicrons, this effect being exacerbated by them competing with VLDL for their common enzymatic system, the LPL /Diabetes Metab, 2005; 31: 429-439/. This leads to an increased accessibility of TGs and FFAs to hepatic cells, essential for VLDL synthesis /Kardiol Pol, 2008; 66: 1215-1220/. Due to an upregulated VLDL concentration in states of insulin resistance, the cumulative apoprotein CIII - inhibiting lipoprotein lipase, and apoprotein C I (apoC I) are also increased. The presence of apoC I inhibits reuptake of the lipoprotein particle by hepatic receptors /J Lipid Res, 1997; 38: 2173-2192/, which aggravates the effect of postprandial hyperlipemia and prolongs the duration of plasma clearance for VLDLs and chylomicrons.

The end products of the degradation of VLDLs and chylomicrons are remnants. The elevated triglyceride content in VLDL remnants (IDLs) and chylomicron remnants (occurring in insulin resistance) induces hepatic lipase (HL), which hydrolyzes TGs in those particles /Diabetes Metab, 2005; 31: 429-439/. Additionally, the triglycerides from remnants may be transported, mediated by a CETP protein (cholesteryl ester and TG transfer protein), between intermediate, low and high density lipoproteins (IDL, LDL and HDL) /Diabetes Metab, 2005; 31: 429-439, Biochim Biophys Acta, 2000; 1529: 257-275/. In states of insulin resistance, the CETP protein is characterized by an upregulated activity and expression, similarly to the abovementioned hepatic lipase. The LDLs and IDLs in turn, being richer in triglycerides, have a longer half-time, with the result that they are available for a longer time for the hepatic lipase. This promotes the conversion of LDLs into small dense LDLs (sd-LDLs), the most atherogenic fraction of lipoproteins /Biochem Soc Trans, 2003; 31:1070-1074/.

The upregulated expression of CETP and hepatic lipase and the downregulated expression of LPL in patients suffering from diabetes also have an adverse effect on the HDL fraction /Diabetes Metab, 2005; 31: 429-439/. First of all, due to the reduced LPL activity, the amount of total cholesterol (CH), apoproteins and phospholipids released in the process of degradation of VLDLs and chylomicrons and employed in HDL biosynthesis process is limited. Secondly, insulin deficiency inhibits the expression of an ATP-binding membrane transporter, which interacts with apoA1 of the HDL lipoprotein in the process of absorbing cholesterol and phospholipids /Diabetes Metab, 2005; 31: 429-439/. In effect, the amount of total cholesterol in a HDL particle is reduced. In turn, the enzyme lecithin:cholesterol esterase, inhibited by insulin deficiency, does not convert cholesterol into esters thereof /Diabetes Metab, 2005; 31:429-439/. Additionally, in insulin resistance, with a CETP overexpression, the reduced pool of cholesterol in HDLs will be subjected to an increased exchange thereof for triglycerides coming from VLDLs. A HDL particle enriched in TGs is degraded faster by the HL, which is taken as the reason for lower levels of these lipoproteins in people with diabetes /Diabetes Metab, 2005; 31: 429-439/.

In conclusion, the long-term disorders in glucose metabolism lead to synthesis of new transcription factors, modifying the expression of numerous genes, including: lipoprotein lipase, the CETP protein, the microsomal transfer protein or HL, among others. Due to this, in patients with type 2 diabetes, the lipid profile is characterized by high TG values, elevated sd-LDL and glyco-LDL levels and a low HDL level. In case of type 1 diabetes, HDL level can be normal or even high, which is a result of a much larger accumulation of TGs in these particles than in other glucose metabolism disorders.

The key to understanding the mechanism of atherosclerotic plaque formation in individuals with carbohydrate turnover disorders is the fact that rather than only LDL also other lipoproteins enriched in apoB100 /Arterioscler Thromb Vasc Biol, 2008; 28:792-797/ permeate into the subendothelial layer of the vessel. Therein, an oxidative transformation of apoprotein B100 occurs /Clin Chimica Acta, 2010; 411: 23-24, 1875-1882, Clin Rev Allerg Immunol, 2009; 37: 4-11/, mediated by its interaction with active aldehydes. The final effect of this process is neutralization of positively charged e-amine groups in lysine /FASEB J, 2001; 15: 2073-2084/ in apoB100. The such modified LDL particles are recognized by scavenging receptors of phagocytic cells /Progress Lipid Res, 2006; 45: 379-404/ present in the vessel wall. In individuals suffering from glucose metabolism disorders, the process of low density lipoprotein oxidation goes more intensively and effects thereof in the form of atherosclerotic plaque synthesis appear much sooner than in the general population. This is associated with an increase in synthesis of oxygen free radicals, an enhanced activation of lipoxygenase and myeloperoxidase and presence of high plasma content of reducing glucose. It is the hyperglycemia which causes the additional glycosylation of LDL, and specifically apoB100 /Diab Vasc Dis Res,2010;7:289-229/. The such modified low density lipoproteins (glyco-LDLs) are no longer recognized by specific cellular receptors (LDLR), while they show affinity to scavenging receptors on macrophages, a ligand therefor being also sd-LDL. It also turns out that glyco-LDLs are able to initiate monocyte migration from the lumen of the vessels to the subendothelial space with identical ease as sd-LDL /Diab Vasc Dis Res,2010; 7: 289-229/. This adversary effect is additionally exacerbated by glycosylation of apoprotein A1 in HDL /Diab Vasc Dis Res,2010; 7:289-229/. The consequence of this process is in turn an impairment in the reverse cholesterol transport. Thus, the enhanced sd-LDL pool found in people with diabetes, supplemented by glyco-LDL, as well as qualitative changes in HDL fraction, seem to explain the increased prevalence of atherosclerotic lesions in these patients.

Overall, the long-term disorders in glucose metabolism lead *inter alia* to an enhanced synthesis of aberrant lipid fractions and adverse transformations thereof, such as oxidation and glycosylation. With oxidation and glycosylation of lipids delivered to the vessel wall being the key step initiating the formation of an atherosclerotic plaque. Glucose intolerance and/or diabetes, in particular resulting from insulin resistance, this being associated directly with obesity, are a problem of contemporary medicine which is enormous in terms of the scale of this process (around 100 million people in Europe suffer from diabetes or glucose intolerance, and furthermore it is estimated that in 20% of the general population excess weight or obesity are observed).

The current state of the art in the field of prevention includes recommendations that in patients with an already diagnosed atherosclerosis or a high risk of occurrence thereof in the future, as well as in people with diabetes, statin preparations should be used first, in order to reduce plasma concentrations of LDL and triglycerides (TGs) /Kardiologia Polska 2016; 74, 11: 1234-1318/.

Lipid disorders, regardless of the type of diabetes, have a similar form /Kardiologia Polska 2016; 74, 9: 821-936/. However, therapy thereof is an extremely complex clinical problem. It should be noted that with a proper control of glycemia most of the quantitative disorders is corrected but the qualitative disorders, namely the upregulated levels of, for example, TGs in VLDLs or LDLs are irreversible. The qualitative changes are a particular concern for clinicians, as such modified non-HDL fractions may undergo faster oxidative transformation in vessel wall. Hence it seems that the obligatory procedure with these patients should indeed be the use of a statin preparation /Kardiologia Polska 2016; 74, 9: 821-936/. The confirmation may be the fact that in patients with type 2 diabetes, in primary prevention of ischemic heart disease (IHD) in multicenter clinical trials with simvastatin (ASCOT-LLA and HPS) or atorvastatin (CARDS), a 30% reduction of prevalence of the first-order endpoint (defined as acute coronary syndromes, coronary revascularization or brain stroke) was shown. The effect was comparable in all of the abovementioned trials, although it was achieved in different time periods from the application of the lipid-lowering treatment /Drugs, 2007; 67 (suppl. 1): 43-54/.

Despite the fact that the mechanism of the pleiotropic effect of large statin doses is not entirely understood, the use thereof may cause regression of atherosclerotic lesions, particularly when the low density lipoprotein levels do not exceed 70 mg/dl (JUPITER) /Lancet, 2009; 373: 1175-1182/.

In subpopulations of patients with type 2 diabetes and/or metabolic syndrome, in GRACE, TNT, or Prove-IT trials, it was shown that in secondary prevention as well, statins decreased total mortality, mortality due to cardiovascular heart diseases, mortality due to coronary disease or brain stroke. This effect was much more pronounced in people with type 2 diabetes or metabolic syndrome in comparison to people without these afflictions and was dependent on the type and dose of the statin used /Open Cardiovasc Med J, 2011; 5: 24-34/. It was also shown that in patients with type 1 diabetes, statins inhibit the tendency towards LDL and VLDL oxidation /Diabetes Metab Res Rev, 2003;19: 478-486/. Nevertheless, due to the very small number of clinical trials using statins in subjects with T1DM, the experts recommend for them to rather have the results of trials performed on patients with type 2 diabetes extrapolated.

The mechanism of statin activity involves the reduction of VLDL and LDL levels by a) increase in LDL catabolism (increase in reuptake of LDL by a specific receptor thereof), b) decrease in endogenous cholesterol synthesis by inhibiting HMG CoA reductase, c) inhibition of apoB100 synthesis /J Lipid Res, 2004; 45: 174-185/. However, it appears that despite using statin preparations, patients with advanced atherosclerosis of coronary vessels are characterized by elevated apoB100 levels relative to total cholesterol. These changes have been observed in the present inventors' own research. This effect may be explained either through a reduction in inhibitory activity of the statin on apoB100 synthesis or through the trend for increased sd-LDL formation in these patients.

The major problem in lipoprotein disorders in people suffering from diabetes is not hypercholesterolemia but atherogenic hyperlipidemia, manifesting through high triglyceride concentrations and low HDL content. Thus, in case of failure to achieve target values of TG concentrations, but after LDL concentrations have been reduced to required levels, it is recommended to include fibrates in the treatment /Kardiologia Polska 2016; 74, 11:1234-1318/. The mechanism of activity for these preparations involves an increase in activity for PPAR-y receptors. This leads to increase in lipolysis and degradation of particles enriched in TGs (lipoprotein lipase activation), as well as increase in apoprotein Al and apoprotein All synthesis. Use of fibrates also contributes to increase in HDL levels and reduction in VLDL and LDL.

When, despite of this procedure, the required triglyceride levels cannot be achieved, it is recommended to use unsaturated omega 3 acids /Kardiologia Polska 2016; 74, 11: 1234-1318/. Positive clinical results are demonstrated however only for ethyl esters of eicosapentaenoic acid (EPA) and docosahexaenoic acid DHA. The mechanism of beneficial activity of EPA and DHA is not fully understood. It is probably associated with inhibition of endogenous lipoprotein synthesis in the liver, as well as with production of eicosanoid precursors, which inhibit platelet aggregation, reduce cytokine and proinflammatory prostaglandin synthesis and stabilize cellular membranes (anti-arrhythmic activity). These properties of ethyl esters of unsaturated omega 3 acids are the reason their use is recommended by The European Cardiology Society for people with severe heart failure (in the GISSI-HF trial beneficial clinical effects of these preparations were demonstrated) Kardiologia Polska 2016; 74, 10: 1037-1147/ and in people with high triglyceride levels /Kardiologia Polska 2016; 74, 11: 1234-1318/. Several years ago, in order to lower the concentration of triglycerides and other lipid fractions, as well as to reduce oxidative stress conditions in patients with atherosclerosis, very large doses of nicotinic acid were also used. Nicotinic acid is a member of the vitamin B group (B3) and is a precursor for two co-enzymes: NAD (nicotinamide adenine dinucleotide) and NADP (nicotinamide adenine dinucleotide phosphate), involved in hydrogen and electron transfer in processes of cellular respiration, glycolysis and lipid biosynthesis.

The key area of nicotinic acid activity is the liver (wherein it inhibits lipogenesis) and fatty tissue (wherein it inhibits lipolysis). In consequence, nicotinic acid reduces the influx of fatty acids to the liver and its secretion of very low density lipoproteins (VLDL). Simultaneously, nicotinic acid has a positive effect on increasing return transport of cholesterol to the liver, through an increase in high density lipoprotein (HDL) and apoA1 protein synthesis. This effect is achieved by using nicotinic acid in large doses of 1500-2000 mg. However, due to the side effects associated with intolerance of such high concentrations of nicotinic acid (allergic reactions, abdominal pains) (the AIM HIGH, HPS2-THRIVE trials) use thereof were discontinued in the general population, with the clinical indications limited to treating particular, very narrow patient groups /Kardiologia Polska 2016; 74, 11: 1234-1318/.

There are only two reports in literature describing clinical effects of combined use of ethyl esters of unsaturated omega 3 acids (EPA and DPA) and nicotinic acid. A very advantageous effect was shown therein of reducing atherogenic lipid fraction levels with this type of therapy /J. Lipid Res. 2012. 53:2429-2435, Cholesterol. 2010/, but nevertheless a large dose of nicotinic acid was used.

The object of the invention is a pharmaceutical composition for reduction of atherogenic lipid fraction levels, oxidative stress and a generally understood atherosclerosis risk, demonstrating clinical efficacy with regard to positive effect on atherogenic lipid fractions, having possible use in manufacture of medicaments and medical supplies intended for primary and secondary prevention of cardiovascular events.

The pharmaceutical composition consists of ingredient A, which is a mixture of ethyl ester of eicosapentaenoic acid (EPA) and ethyl ester of docosahexaenoic acid (DHA) in mass ratio of 1 : 0.6 ÷ 1.0, and ingredient B, which is nicotinic acid amide, in mass ratio to the ethyl ester of eicosapentaenoic acid (EPA) of 1 : 0.1 ÷ 2, within a daily dose range for nicotinic acid amide between 25 mg - 500 mg, for simultaneous administration.

Preferably, ingredient A and ingredient B are intended for sequential administration.

Preferably, ingredient A and ingredient B are intended for administration in a unit dose form.

Preferably, the mass ratio of the ethyl ester of eicosapentaenoic acid (EPA) and the ethyl ester of docosahexaenoic acid (DHA) is 1 : 0.817.

The object of the invention is also a pharmaceutical composition comprising ingredient A, which is a mixture of ethyl ester of eicosapentaenoic acid (EPA) and ethyl ester of docosahexaenoic acid (DHA) in mass ratio of 1 : 0.6 ÷ 1.0, and ingredient B, which is nicotinic acid amide, in mass ratio to the ethyl ester of eicosapentaenoic acid (EPA) of 1 : 0.1 ÷ 2, within a daily dose range for nicotinic acid amide between 25 mg - 500 mg , as a combined preparation for primary and secondary prevention of cardiovascular events.

Preferably, ingredient A and ingredient B are intended for sequential administration.

Preferably, ingredient A and ingredient B are intended for administration in a unit dose form.

Preferably, the mass ratio of ethyl ester of eicosapentaenoic acid (EPA) and ethyl ester of docosahexaenoic acid (DHA) is 1 : 0.817.

Nicotinic acid amide is a derivative of nicotinic acid, differing in the presence of a substituent (amide group) at the C1 carbon. It turns out that it is in this form that the compound retains advantageous therapeutic properties of nicotinic acid, without simultaneously showing adverse side effects thereof caused by the interaction of niacin with a nicotine receptor, *inter alia* in skin, with a significantly reduced daily dose. It was unexpectedly found that the desired clinical effect of reduction in the levels of triglycerides and other lipid fractions was achieved due to synergistic use of routine doses of ethyl esters of eicosapentaenoic acid (EPA) and docosahexaenoic acid (DHA) and very low doses of nicotinic acid amide, those being 75%-95% lower than the doses of nicotinic acid used in clinical trials. Moreover, such a combination of substances has never been used before together in the context of prevention of atherosclerosis. According to the invention it was found that replacing nicotinic acid with its amide enables lowering the dose, this providing an advantageous reduction in undesired side effects, while at the same time unexpectedly providing a therapeutic effect analogous to the one obtained with administering much higher doses of nicotinic acid.

The composition being the object of the invention is characterized by high clinical safety, while simultaneously demonstrating clinical efficacy in the context of an advantageous effect on atherogenic lipid fractions, which may find use in manufacturing of medicaments and medical supplies intended for primary and secondary prevention of cardiovascular events.

### Example.

The effect of the therapeutic composition of compounds of ethyl esters of eicosapentaenoic acid (EPA) and docosahexaenoic acid (DHA) combined with nicotinic acid amide, in mass ratio of 1 : 0.6 ÷ 1.0 : 0.1 ÷ 2 respectively, in a narrow range of doses for nicotinic acid amide between 25 mg - 500 mg, was evaluated in patients in primary and secondary prevention of cardiovascular events, using routine laboratory methods determining the levels for lipid profile (TC, HDL, LDL, TG), homocysteine, as well as in some cases the levels of aminotransferases and creatinine kinase. The composition according to the invention was used to supplement fibrate and/or statin therapy in a group of people having very high cardiovascular risk and in several individuals who did not consent for fibrate/statin use (patient 19) or had contraindications against using fibrates/statins (patient no. 3, 12). The use of ethyl esters of eicosapentaenoic acid (EPA) and docosahexaenoic acid (DHA) combined with nicotinic acid amide, in mass ratio of 1:0.6-1.0:0.1-2 /but always within a narrow range of doses for nicotinic acid amide between 25 mg - 500 mg/ was free from any undesired effects (in particular those reported in the context of using nicotinic acid). The composition was used twice daily in the following dose range: for EPA from 460 mg to 2300 mg per day, for DHA from 380 mg to 1920 mg per day, for nicotinic acid amide 25 - 500 mg per day. The medications were used combined, two or three times a day or in a unit dose form. Time delay between using one substance and another was inconsequential, and likewise there was no relationship or lack thereof with the meal taken. The effect of the composition was studied for 6 weeks (+/- 2). The effect of the aforementioned therapeutic composition was not studied in a group of healthy subjects due to the fact that according to the declaration of Helsinki this would require getting approval from a bioethics committee for subjecting such a population to a medical experiment. Thus, a comparison of the therapeutic effect of the aforementioned therapeutic composition in a group of subjects with lipid disorders relative to healthy subjects was not possible.

The doses used in patients 1-21 are shown in table 1.

**Table 1.**

| Patient no. | EPA and DHA esters, dose per day in mg, ratio of EPA to DHA is 1:0.817 | Nicotinic acid amide, dose per day in mg | Statin, dose per day in mg | Fibrate, dose per day in mg |
|---|---|---|---|---|
| 1 | 854 | 100 | 13.3 | 0 |
| 2 | 1708 | 100 | 40 | 267 |
| 3 | 2562 | 100 | 0 | 0 |
| 4 | 1708 | 100 | 40 | 215 |
| 5 | 1708 | 100 | 20 | 215 |
| 6 | 2562 | 200 | 40 | 215 |
| 7 | 1708 | 100 | 30 | 215 |
| 8 | 2562 | 200 | 20 | 215 |
| 9 | 2562 | 100 | 40 | 267 |
| 10 | 3416 | 200 | 40 | 0 |
| 11 | 2562 | 100 | 40 | 160 |
| 12 | 2562 | 100 | 30 | 215 |
| 13 | 2562 | 100 | 10 | 0 |
| 14 | 2562 | 200 | 40 | 215 |
| 15 | 2562 | 100 | 10 | 145 |
| 16 | 1708 | 100 | 20 | 145 |
| 17 | 2562 | 100 | 40 | 215 |
| 18 | 2562 | 200 | 0 | 0 |
| 19 | 2562 | 100 | 40 | 267 |
| 20 | 2562 | 200 | 40 | 267 |
| 21 | 854 | 100 | 40 | 160 |

The results obtained are shown in table 2.

**Table 2**

| Patient no. | TG1 [mmol/L] | TG2 [mmol/L] | LDL1 [mmol/L] | LDL2 [mmol/L] | TC1 [mmol/L] | TC2 [mmol/L] | HDL1 [mmol/L] | HDL2 [mmol/L] | Homocysteine [ug/L]; Normal values up to 16 | Side effects |
|---|---|---|---|---|---|---|---|---|---|---|
| 1 | 2.2 | 1.5 | 2.5 | 1.9 | 4.9 | 3.9 | 1.4 | 1.4 | 10.43 | 0 |
| 2 | 3.1 | 2.4 | 3.8 | 3.6 | 6.3 | 5.8 | 1.1 | 1.1 | 18.38 | 0 |
| 3 | 16 | 3.3 | | 2.1 | 6.8 | 4.8 | 0.4 | 1.2 | 10.2 | 0 |
| 4 | 7.9 | 5.2 | | 5.4 | 8.2 | 9.2 | 1.2 | 1.5 | 17 | 0 |
| 5 | 38 | 2.4 | | 1.1 | 26.8 | 3 | | 0.8 | 12.68 | 0 |
| 6 | 18.2 | 9.6 | | | 7.3 | 3.4 | 0.5 | 0.4 | 19 | 0 |
| 7 | 2.3 | 1.8 | 1.2 | 1.8 | 3.3 | 3.7 | 1.1 | 1.1 | 11.5 | 0 |
| 8 | 7.62 | 3.19 | | 1.68 | 5.27 | 3.5 | 0.85 | 0.98 | | 0 |
| 9 | 2.6 | 1.1 | 2.5 | 1.7 | 5.2 | 3.9 | 1.5 | 1.7 | 15 | 0 |
| 10 | 5.95 | 2.8 | | 2.7 | 6.67 | 4.9 | 0.77 | 0.9 | 12.3 | 0 |
| 11 | 14.1 | 2 | | 1.3 | 14.8 | 4.2 | 1.7 | 2 | 21 | 0 |
| 12 | 3 | 2.5 | 1.6 | 1.9 | 3.7 | 3.8 | 0.8 | 0.8 | 7.95 | 0 |
| 13 | 3.3 | 2.5 | 2.7 | 2.6 | 5.3 | 4.8 | 1.1 | 1.1 | 8.08 | 0 |
| 14 | 2.5 | 3 | 2.3 | 1.1 | 4 | 3.4 | 1 | 1 | 9.73 | 0 |
| 15 | 2.8 | 1.5 | 2.6 | 1.3 | 4.8 | 3.1 | 0.9 | 1.1 | 16.5 | 0 |
| 16 | 2.8 | 2.3 | 2.1 | 1.1 | 4.6 | 3.5 | 1.1 | 1.3 | 14 | 0 |
| 17 | 14.3 | 4.1 | | 0.7 | 5.7 | 3 | 0.5 | 0.4 | 15 | 0 |
| 18 | 11.3 | 3.35 | 7.37 | 115 | | 5.4 | 0.66 | 35 | 9.32 | 0 |
| 19 | 8.03 | 1.2 | 2.95 | 1.1 | 7.68 | 3.2 | 1.04 | 1.6 | | 0 |
| 20 | 8.9 | 9.9 | | | 5.22 | 5.8 | 0.46 | 0.2 | 26 | 0 |
| 21 | 14.9 | 7.2 | 4.44 | | 10.2 | 5.5 | 1.13 | 0.8 | 10.7 | 0 |

| | | | | | | | | | | |
|---|---|---|---|---|---|---|---|---|---|---|
| Note: no LDL levels in some columns is a consequence of the measurement methodology for this parameter. | | | | | | | | | | |

If TG >4 mmol/L then calculation of LDL level becomes impossible except when with a method of direct measurements for LDL, which was the case for patient no. 21.

Reference 1 indicates results before treatment, reference 2 indicates results after treatment.

The results of laboratory studies summarized in Table 2 demonstrate the efficacy in terms of reduction of atherogenic lipid fraction levels based on supplementing routine lipid-lowering treatment with the compounds being the object of the present application. At the same time, information concerning the lack of undesired effects while using the abovementioned therapy is shown.

## Claims

1. A pharmaceutical composition with ethyl ester of eicosapentaenoic acid (EPA) and ethyl ester of docosahexaenoic acid (DHA), **characterized in that** it consists of ingredient A, which is a mixture of ethyl ester of eicosapentaenoic acid (EPA) and ethyl ester of docosahexaenoic acid (DHA) in mass ratio of 1 : 0.6 ÷ 1.0, and ingredient B, which is nicotinic acid amide, in mass ratio to the ethyl ester of eicosapentaenoic acid (EPA) of 1 : 0.1 ÷ 2, within a daily dose range for nicotinic acid amide between 25 mg - 500 mg, for simultaneous administration.

2. The composition according to claim 1, **characterized in that** ingredient A and ingredient B are intended for sequential administration.

3. The composition according to claim 1, **characterized in that** ingredient A and ingredient B are intended for administration in a unit dose form.

4. The composition according to claim 1 or 2 or 3, **characterized in that** the mass ratio of the ethyl ester of eicosapentaenoic acid (EPA) and the ethyl ester of docosahexaenoic acid (DHA) is 1 : 0.817.

5. A pharmaceutical composition comprising ingredient A, which is a mixture of ethyl ester of eicosapentaenoic acid (EPA) and ethyl ester of docosahexaenoic acid (DHA) in mass ratio of 1 : 0.6 ÷ 1.0, and ingredient B, which is nicotinic acid amide, in mass ratio to the ethyl ester of eicosapentaenoic acid (EPA) of 1 : 0.1 ÷ 2, within a daily dose range for nicotinic acid amide between 25 mg - 500 mg , as a combined preparation for primary and secondary prevention of cardiovascular events.

6. The composition according to claim 5, **characterized in that** ingredient A and ingredient B are intended for sequential administration.

7. The composition according to claim 5, **characterized in that** ingredient A and ingredient B are intended for administration in a unit dose form.

8. The composition according to claim 5 or 6 or 7, **characterized in that** the mass ratio of the ethyl ester of eicosapentaenoic acid (EPA) and the ethyl ester of docosahexaenoic acid (DHA) is 1 : 0.817.
